Europäisches Patentamt

(19) European Patent Office

Office européen des brevets

(11) Publication number: **0 099 662**

**A1**

(12) # EUROPEAN PATENT APPLICATION

(21) Application number: **83303689.0**

(22) Date of filing: **27.06.83**

(51) Int. Cl.³: **A 61 N 1/36**
**A 61 H 39/00**

(30) Priority: **15.07.82 GB 8220547**

(43) Date of publication of application:
**01.02.84 Bulletin 84/5**

(84) Designated Contracting States:
**AT BE CH DE FR GB IT LI LU NL SE**

(71) Applicant: **Wilmot, John Kenneth**
**4 Ennerdale Close**
**Clitheroe Lancashire(GB)**

(72) Inventor: **Wilmot, John Kenneth**
**4 Ennerdale Close**
**Clitheroe Lancashire(GB)**

(74) Representative: **Oliver, Roy Edward et al,**
**POLLAK MERCER & TENCH High Holborn House 52-54**
**High Holborn**
**London WC1V 6RY(GB)**

(54) Transcutaneous electrical nerve stimulation apparatus.

(57) Transcutaneous electrical nerve stimulation apparatus comprising a random pulse rate generator (7) arranged to provide random frequency, electrical output pulses. Preferably, the random pulse rate generator (7) also provides the output pulses with random durations, whereby the rate of change of frequency of the output pulses is random also. The pulses may be of a square waveform or spiked configuration and the apparatus can also be provided with an override for supplying pulses of a predetermined maximum amplitude which is adjustable.

EP 0 099 662 A1

-1-

# TRANSCUTANEOUS ELECTRICAL NERVE STIMULATION APPARATUS

## DESCRIPTION

This invention relates to transcutaneous electrical nerve stimulation apparatus, sometimes known as electrical acupuncture apparatus but referred to herein as TENS apparatus.

Many known forms of TENS apparatus are commercially available and generally comprise a pulse generator having a controlled frequency and/or amplitude facility, whereby the electrical pulses applied to the skin of a patient, via electrodes, can be suitably varied in dependence upon the nature of the pain suffered by the patient, the physic of the patient and other relevant parameters.

Classical acupuncture is effected by inserting needles into nerves and is believed to block-off or divert the natural electrical pulses which are the normal signals transmitted to the brain to indicate that pain is occurring. The success of acupuncture is based almost totally upon the skill of the operative, whereas TENS apparatii have much the same effect by blanketing the relevant areas under which the nerves are located with a series of voltage pulses, preferably spiked pulses, some of which are bound to be of the correct shape, frequency, location and amplitude to have the desired blocking, diverting or confusing effect upon the natural electrical signals being transmitted to the brain by the nerves in question.

Any of the known types of TENS apparatus is likely to have a beneficial effect, although this is likely to be reduced after a certain time, because the pulses are bound to be generated in accordance with a pattern or code. Indeed, it is thought that the brain will eventually counteract the blocking, diverting or confusing effect by ascertaining the code of the pattern of pulses and thus minimising the acupuncture effect. This blocking, diverting or confirming effect of the pulses is thus reduced and the sensation of pain is no longer suppressed, this becoming evident again.

Additionally, the particular frequency and/or width of the pulses for a given type and location of pain is fairly crucial for suppressing the pain but with known TENS apparatii the exact pulse width and/or frequency is rather a hit-and-miss affair, more often than not the correct pulse width and/or frequency never being achieved.

An object of the present invention is to eliminate, or at least substantially eliminate, the disadvantages described above in relation to known types of TENS devices.

Accordingly, one aspect of the invention provides a TENS apparatus wherein the output pulses are generated at random frequencies and, optionally, for random durations.

Thus, for both random frequency and duration of the output pulses, the rate of change of frequency is also random. Preferably, the pulses are of a generally square waveform configuration but, more preferably, are spiked. Additionally, the amplitude of the pulses can be varied, preferably, up to a maximum of, say, 90 volts.

Another aspect of the invention provides an electrical circuit for generating pulses, comprising a random pulse-rate generator whose output is fed to a step-up transformer via a dc amplifier, the pulsed output of the transformer optionally being applied across a reverse polarity switch.

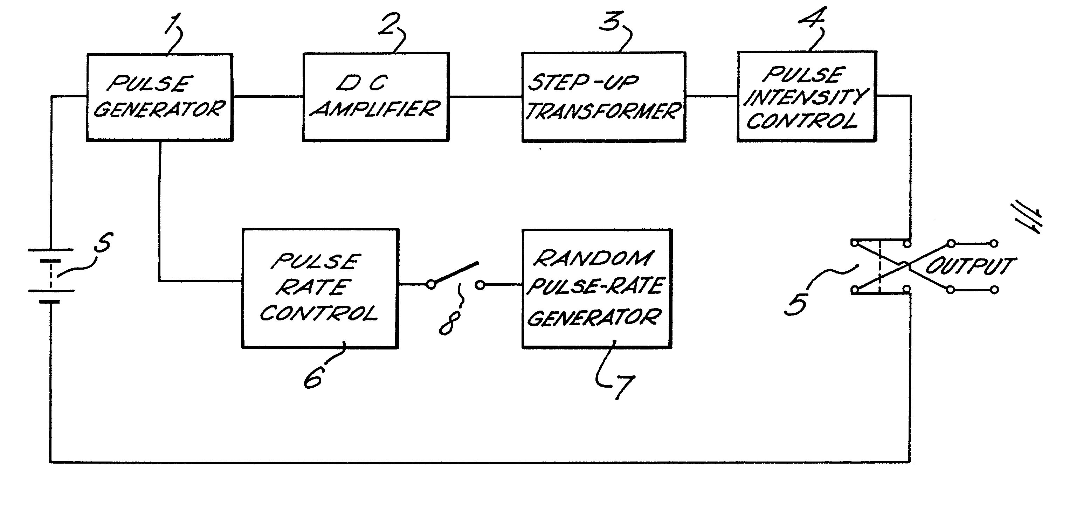

In order that the invention may be more fully understood, a preferred embodiment in accordance therewith, will now be described by way of example and with reference to the accompanying drawing which shows the electrical circuit, in block diagrammatical form, for generating the random frequency pulses.

Referring now to the drawing, the circuit is enclosed within a housing which is sufficiently small to be carried in the pocket of a user. The circuit itself comprises a power source S of 2 x 1½ volt batteries connected to a pulse generator 1 which is connected in series with a dc amplifier 2, a step-up transformer 3 and a pulse intensity control 4. The output of the intensity control 4 is connected to one side of a reverse polarity switch 5, whilst the negative side of the 1½ volt power source S is connected in series with the other side of the control 4.

To the pulse generator 1 is connected a pulse rate control 6 which, in turn, is connected in series with a random pulse-rate generator 7, via a random select switch 8.

The pulsed circuit output from the reverse polarity switch 5 can be applied to the skin of a patient by means of at least one pair of electrodes (not shown), the impedance between the electrodes and the patient's skin being reduced substantially by means of a conductive gel or grease applied therebetween.

The pulse generator 1 may be either an astable multivibrator, with extremely short pulse duration, or a unijunction transistor oscillator. In either case, however, it is desirable to produce a voltage pulse or spike waveform. The frequency of such pulses or spikes is governed by the pulse rate control section of the circuit, which is constituted by the pulse rate control 6 and, optionally, the random pulse-rate generator 7 when the random select switch 8 is closed.

The output of the pulse generator 1 is fed, via the dc amplifier 2, to the step-up transformer 3 whose turns ratio is such that a maximum of 90 volts per pulse amplitude can be generated.

The pulse intensity control 4 provides the means for adjusting the amplitude of the output pulse from the switch 5, in dependence upon the patient's individual requirements. The circuit output pulses from the reverse polarity switch 5,which is shown wired as a double pole changeover switch,are fed to a terminal socket (not shown) for receiving the electrode leads. This switching arrangement provides a polarity reversal feature at the flick of a switch, rather than having to remove manually the terminal pins of the electrode leads and then replace them in the opposite polarity sockets.

The random pulse rate generator 7 can be brought into circuit when the random select switch is closed, with the generator 7 acting directly upon the pulse rate control circuit and causing the random frequency changes to occur. The rate of change of frequency, and the various frequencies generated, within a particular frequency range, thus appear as a train of random frequencies at varying time intervals.

The circuit may be provided with optional monitoring means, such as, a light emitting diode (LED), which is activated in correspondence with the pulsed circuit output. Thus, as the output frequency is randomised, the LED will flash on and off but at higher frequencies it will appear to remain on, due to its being undetectable by human eye response.

As indicated above, the amplitude of the random frequency pulses can be adjusted and, also, it will be appreciated that the circuit can be operated in a standard mode wherein the pulse frequency is constant. Furthermore, it is to be understood that the whole electrical circuit can be mounted within a pocket-size housing for ease of handling and transportation purposes.

The range of frequencies used can be selected according to the particular application of the apparatus. However, for the embodiment described above the preferred frequency range is 1.5 to 500 Hz.

In addition to the digital randomising feature, the circuit may also include an amplitude override control especially designed for obstetric practice.

This override takes the form of a "panic button" attached to one end of a flying lead, with the other end of the lead being plugged into the TENS unit.

During childbirth, the patient may receive the benefits of normally applied TENS until such time as a painful contraction is in evidence. At this point, she may press the override button and increase the amplitude of the TENS pulses. Under normal conditions, this amplitude setting would be considered to be uncomfortable but, during contractions, this would not be so noticeable and the patient would experience the benefit of the increased, pain-blocking effect.

The amount by which the "normal" setting can be increased is adjustable, and can be pre-set by the obstetrician. This adjustment control can be positioned within the "panic button" assembly in such a way as to prevent the patient from accidentally altering the setting. This feature may be provided on one or more channels of a TENS unit.

CLAIMS

1. Transcutaneous electrical nerve stimulation apparatus comprising a pulse rate generator (7),
        characterised in
        that the pulse rate generator (7) is arranged to provide random frequency, electrical output pulses.

2. Apparatus according to claim 1 including means arranged to provide the output pulses with random durations, whereby the rate of change of frequency of the output pulses is also random.

3. Apparatus according to claim 1 and claim 2, wherein said means is constituted by the random pulse rate generator (7).

4. Apparatus according to claim 1,2 or 3, wherein the random pulse rate generator (7) is arranged to provide output pulses of square waveform or spiked configuration.

5. Apparatus according to any preceding claim including means (4) arranged to vary the amplitude of the output pulses.

6. Apparatus according to claim 5, wherein said pulse amplitude varying means (4) is actuable to provide pulses of a predetermined maximum amplitude which is greater than that of the output pulses during normal operation of the apparatus.

7. Apparatus according to claim 6, wherein the predetermined maximum pulse amplitude of said pulse amplitude varying means (4) is adjustable.

8. Apparatus according to any preceding claim including a plurality of outputs providing a plurality of respective random frequency, electrical output pulses.

9. An electrical circuit arrangement, when used with a transcutaneous electrical nerve stimulation apparatus according to any preceding claim, comprising a step-up transformer (3) to which is connected the output of the random pulse rate generator (7) via a dc amplifier (2).

10. A circuit arrangement according to claim 9, wherein the pulsed output of the step-up transformer (3) is applied across a reverse polarity switch (5).

PULSE
GENERATOR
1

D C
AMPLIFIER
2

STEP-UP
TRANSFORMER
3

PULSE
INTENSITY
CONTROL
4

5

PULSE
RATE
CONTROL
6

8

RANDOM
PULSE-RATE
GENERATOR
7

5

OUTPUT

Fig. 1

0099662

European Patent
Office

**EUROPEAN SEARCH REPORT**

Application number

| | DOCUMENTS CONSIDERED TO BE RELEVANT | | EP 83303689.0 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| X | <u>EP - A1 - 0 033 643</u> (MEDTRONIC) <br> * Page 5, lines 16-28; page 8, claim 10 * <br> -- | 1-4 | A 61 N   1/36 <br> A 61 H 39/00 |
| A | <u>DE - A1 - 2 443 913</u> (PITTERLING) <br> * Page 6, lines 9-16,27 - page 7, line 16 * <br> -- | 2,5-10 | |
| A | <u>AT - B - 352 866</u> (LOHER) <br> * Totality * <br> -- | | |
| A | <u>GB - A - 1 546 089</u> (LIM BOON CHEN) <br> * Page 1, line 39 - page 2, line 12 * <br> -- | | |
| A | <u>US - A - 3 817 254</u> (MAURER) <br> * Totality * <br> ---- | | TECHNICAL FIELDS SEARCHED (Int. Cl. ³) <br><br> A 61 N <br> H 03 K <br> A 61 H |

The present search report has been drawn up for all claims

| Place of search | Date of completion of the search | Examiner |
|---|---|---|
| VIENNA | 03-10-1983 | NEGWER |